# EUROPEAN PATENT SPECIFICATION

(11) **EP 1 713 391 B1**
(45) Date of publication and mention of the grant of the patent: **26.11.2008**
(21) Application number: 05712899.3
(22) Date of filing: 04.02.2005
(51) Int. Cl.: A61B 5/15

(54) **DAMPENING AND RETRACTION MECHANISM FOR A LANCING DEVICE**
DÄMPFUNGS- UND RETRAKTIONSMECHANISMUS FÜR EINE LANZETTENVORRICHTUNG
MECANISME AMORTISSEUR ET DE RETRAIT POUR UN DISPOSITIF PERFORANT

(30) Priority: 06.02.2004 US 542779 P
(43) Date of publication of application: 25.10.2006
(73) Proprietor: Bayer HealthCare LLC, Tarrytown, NY 10591 (US)
(72) Inventor: PURCELL, Glenn, Edwardsburg, Michigan 49112 (US)
(74) Representative: Linhart, Angela
(86) International application number: PCT/US2005/003623
(87) International publication number: WO 2005/077275

(56) References cited:
- EP-A- 0 898 936
- US-A- 4 517 978
- US-A1- 2002 022 789

## Description

### FIELD OF THE INVENTION

The present invention relates generally to diagnostic instruments and, more particularly, to a system for repositioning a moveable housing and damping a lancet utilizing a secondary spring.

### BACKGROUND OF THE INVENTION

The quantitative determination of analytes in body fluids is of great importance in the diagnoses and maintenance of certain physiological abnormalities. For example, lactate, cholesterol and bilirubin should be monitored in certain individuals. In particular, determining glucose in body fluids is important to diabetic individuals who must frequently check the glucose level in their body fluids to regulate the glucose intake in their diets.

One method of obtaining a body fluid sample such as a whole blood sample is to use a lancing device. The whole blood sample may be used to monitor the glucose of an individual. Existing lancing devices use a lancet to pierce the tissue of the skin, allowing a blood sample to form on the skin's surface. The whole blood sample is then transferred to the testing device. The whole blood sample is often taken from the fingertips of a test subject for glucose monitoring because of the high concentration of capillaries that can provide an effective blood supply. Taking the blood from the fingertips, however, is disadvantageous because of the high concentration of nerve endings that cause pain and discomfort to many individuals.

In addition to the pain and discomfort inherent in piercing the fingertip, existing lancing devices may cause increased pain to many individuals by failing to properly dampen the lancet after initially piercing the skin. This may result in multiple punctures to the individual's skin, requiring additional healing time and increasing the discomfort to the user. Alternatively, excessive damping can reduce the lancet's force and adversely effect the puncture depth, causing insufficient sample size and the need to lance again.

It would be desirable to have a lancing device and method that addresses these issues while reducing the number of components required to manufacture the lancing device, and thus, reducing the overall cost of the device.

Documents EP 0898936 and US 4517978 both disclose a blood sampling device with a moveable housing and springs for cocking the device.

### SUMMARY OF THE INVENTION

According to one embodiment of the present invention a lancing device is disclosed. The lancing device comprises a main housing having an internal surface enclosing a portion of a lancing mechanism. The lancing mechanism includes a lancet holder attached to a shaft and a drive spring surrounding a portion of the shaft. The drive spring is located between the lancet holder and the internal surface. The lancing mechanism is adapted to move between a resting position, a cocking position, and a puncture position. The lancing device further comprises a movable housing adjacent the main housing. The movable housing is adapted to move from a resting position to a cocking position. The moveable housing has an internal surface enclosing a portion of the shaft of the lancing mechanism. The enclosed portion of the shaft has a retainer and a secondary spring surrounding at least a section of the shaft. The secondary spring is located between the retainer and the internal surface of the movable housing. The secondary spring is adapted to move the movable housing from the cocking position to the resting position. The secondary spring is further adapted to move the lancing mechanism from the puncture position to the resting position.

According to an example, a method for damping a lancet utilizing the above-described lancing device is disclosed. The method includes the acts of providing the above-described lancing device and compressing the drive spring and the secondary spring by moving the movable housing away from the main housing to the cocking position. The method further includes the acts of decompressing the secondary spring to move the movable housing from the cocking position to the resting position, adjacent the main housing and actuating the drive spring to cause the lancet holder to move from the cocking position to the puncture position. The method further comprises the acts of recompressing the secondary spring as the lancet holder moves from the cocking position to the puncture position and decompressing the secondary spring to move the lancet holder from the puncture position to the resting position.

The above summary of the present invention is not intended to represent each embodiment, or every aspect, of the present invention. This is the purpose of the Figures and the detailed description which follow.

### BRIEF DESCRIPTION OF THE DRAWINGS

The foregoing and other advantages of the invention will become apparent upon reading the following detailed description and upon reference to the drawings.

FIG. 1 is a perspective view of a lancing device and endcap, according to one embodiment of the present invention.

FIG. 2 is a front view of the lancing device of FIG. 1.

FIG. 3 is a front view of the lancing device of FIG. 1 with a lancet received therein.

FIG. 4 is a cross-sectional view of the lancing device of FIG. 1 with a lancet received therein, the lancing device being in a resting position.

FIG. 5 is a cross-sectional view of the lancing device of FIG. 1 in a cocking position.

FIG. 6 is a cross-sectional view of the lancing device of FIG. 1 in a cocked position.

FIG. 7 is a cross-sectional view of the lancing device of FIG. 1 in a puncture position.

FIG. 8 is a front view of a pushbutton of the lancing device of FIG. 1, according to one embodiment of the present invention.

FIG. 9 is a perspective view of a lancing mechanism contained within the lancing device of FIG. 1, according to one embodiment of the present invention.

While the invention is susceptible to various modifications and alternative forms, specific embodiments have been shown by way of example in the drawings and will be described in detail herein. It should be understood, however, that the invention is not intended to be limited to the particular forms disclosed. Rather, the invention is to cover all modifications, equivalents, and alternatives falling within the scope of the invention as defined by the appended claims.

### DESCRIPTION OF ILLUSTRATIVE EMBODIMENTS

The present invention is directed to a lancing device that is adapted to receive a lancet for use in drawing a body fluid from a test subject. The body fluid generally contains at least one analyte that may then be examined to determine its concentration in the body fluid sample.

Lancing devices and lancets may be used to produce a blood or body fluid sample from a test subject. This sample may then be analyzed with a meter and test strip, or similar devices, to determine the concentration of the analyte to be examined. Examples of the types of analytes that may be collected with a lancing device include glucose, lipid profiles (*e.g.*, cholesterol, triglycerides, LDL and HDL), microalbumin, hemoglobin A1C, fructose, lactate, or bilirubin.

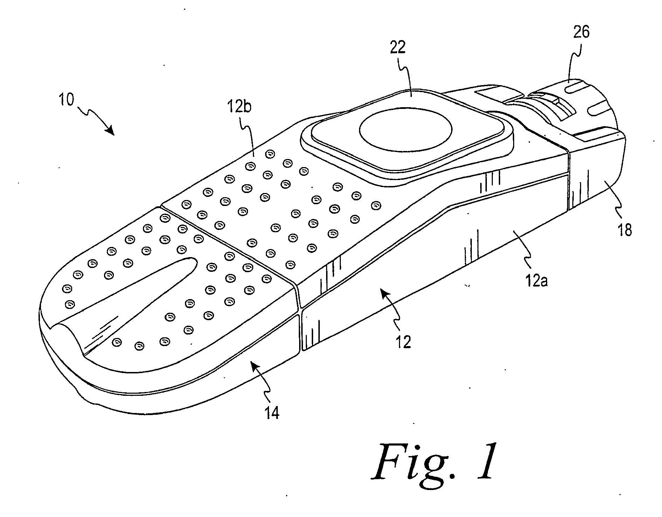

Turning now to the drawings and initially to FIGS. 1-3, a lancing device 10 for obtaining a fluid sample from a test subject is illustrated, according to one embodiment of the present invention. The lancing device 10 has a main housing 12 with a movable housing 14 movable relative to the main housing 12. The main housing 12 includes a first main housing portion 12a and a second main housing portion 12b. The first and second main housing portions 12a,b may be removeably attachable or may be formed or molded as one permanently attached piece. An end cap support 16 is connected to the main housing 12 on the testing end of the lancing device 10. An end cap 18 may be removably attached to the end cap support 16. When attached, the end cap 18 is retained on the end cap support 16 by, for example, a pair of support arms 20a-b integrally formed with the end cap support 16.

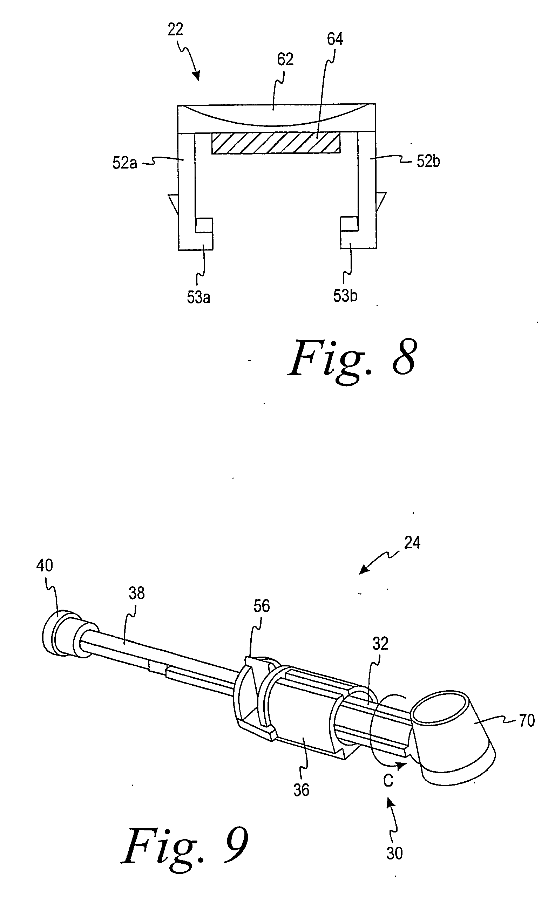

When used, the movable housing 14 is pulled away from the main housing 12 to move an internal lancing mechanism to a cocked position, and then a pushbutton 22 is depressed to actuate the lancing mechanism 24 (FIG. 9) so that a sharp tip of a lance 34 of a lancet 30 is forced through an aperture (not shown) formed in the end cap 18. The lancing device 10 may be provided with a number of different end caps 18, each having a different width, to facilitate the formation of skin punctures of various depths. Alternatively, the end cap 18 may include an adjustable dial 26 for allowing punctures of different depths to be performed utilizing a single end cap 18.

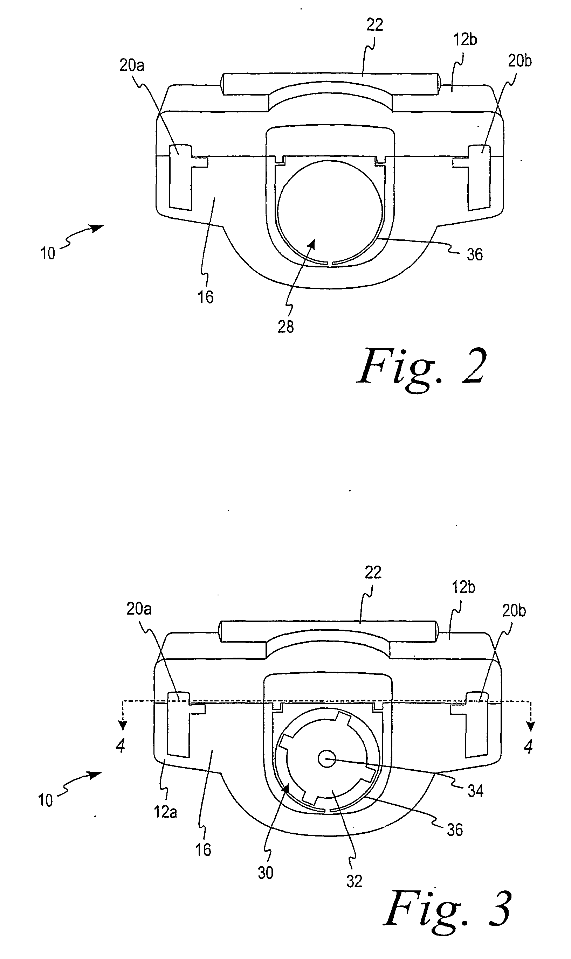

FIGS. 2-3 illustrate the lancing device 10 with the endcap 18 removed. The lancet holder 36 includes a central, generally-cylindrical aperture 28 formed therein. The aperture 28 is adapted to receive the lancet 30, as illustrated in FIG. 3. The lancet 30 includes a lancet body 32 with a sharp-tipped lance 34 extending therefrom. The lance 34 may be enclosed within a protective cap 70 (FIG. 9) to protect a user from unintended punctures. Additionally, the protective cap 70 assists with preventing the lance 34 from being contaminated prior to use and also may be replaced after the use of the lance 34, prior to discarding the lancet 30.

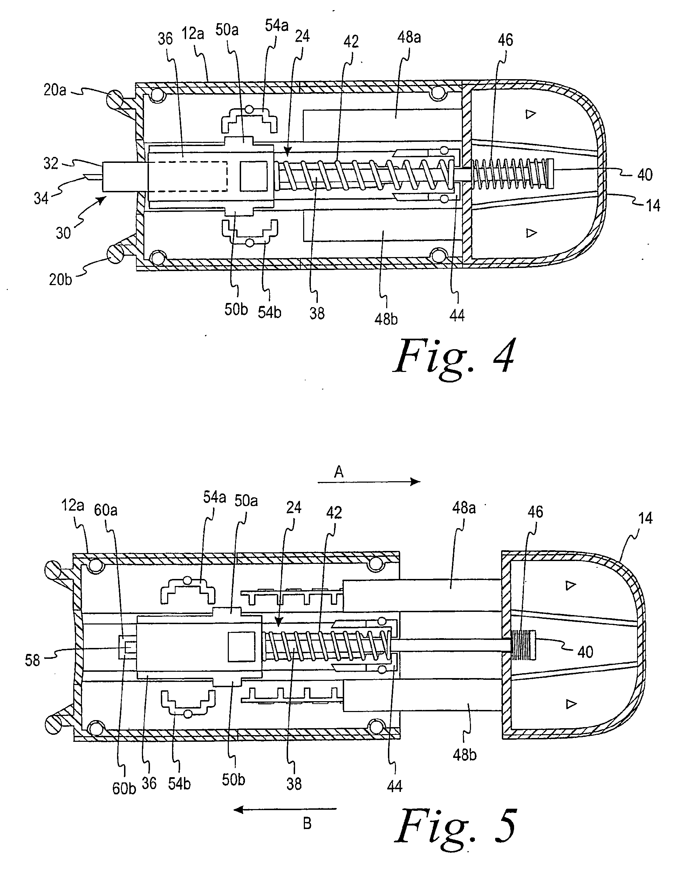

Referring also to FIG. 4, a cross-sectional view of the lancing device 10 in a resting position with the end cap 18 detached is illustrated. The lancet holder 36 is connected to an elongated shaft 38 by being integrally formed therewith. The shaft 38 has an retainer 40 that is supported within the movable housing 14. A drive spring 42 is disposed around the shaft 38 between the lancet holder 36 and a spring stop 44 integrally formed with the first main housing portion 12a.

The movable housing 14 has a pair of elongated columns 48a,b integrally formed therewith. Each of the columns 48a,b extends into the main housing 12 through an aperture (not shown) formed in the first main housing portion 12a. A secondary spring 46 is disposed around the shaft 38 within the movable housing 14. A first end of the secondary spring 46 is disposed against an internal surface of the moveable housing 14 and a second end of the secondary spring 46 is disposed against the retainer 46 of the shaft 38. The secondary spring 46 is centrally located within the movable housing 14 along the longitudinal axis of the lancing device 10.

FIG. 4 illustrates the interior of the lancing device 10 when the lancing device 10 is not in use. In this position, the lancet holder 36 is disposed in a resting position between a puncture position and a cocked position. In the resting position, both the drive spring 42 and the secondary spring 46 are substantially uncompressed and are in equilibrium with each other. FIG. 5 illustrates the interior of the lancing device 10 (the lancet 30 is not shown) when the lancet holder 36 and movable housing 14 are in a cocking position in which the movable housing 14 has been pulled away from the main housing 12. In the cocking position, both the drive spring 42 and the secondary spring 46 are substantially compressed as the user moves the movable housing 14 away from the housing 12 in the direction of Arrow A.

Referring now to FIGS. 4-6 and FIG. 8, to move the lancet holder 36 from its resting position to its cocked position, the movable housing 14 is pulled away from the main housing 12 in the direction of Arrow A. The movable housing 14 continues to be pulled-against the force of the drive spring 42 and the secondary spring 46-until a plurality of angled stop members 50a,b formed on the lancet holder 36 move past (to the right of as illustrated in FIGS. 4-6) a plurality of catch arms 52a,b located on the pushbutton 22 (as best illustrated in FIG. 8). Each of the catch aims 52a,b has a respective end 53a,b adapted to engage the angled stop members 50a,b. The ends 53a,b of the catch arms 52a,b are angled opposite the angled stop members 50a,b, such that when the angled stop members 50a,b are moved in the direction of Arrow A they contact the ends 53a,b of the catch arms 52a,b. The movement of the angled stop members 50a,b force the ends 53a,b of the catch arms 52a,b-as well as the attached pushbutton 22-in the direction of the first main housing portion 12a.

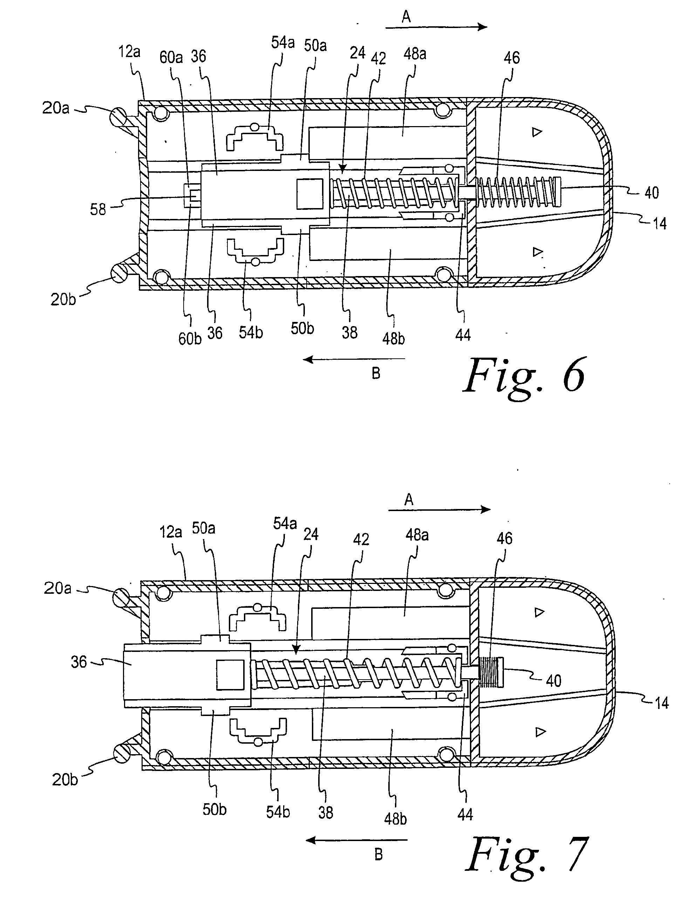

Once the angled stop members 50a,b have moved past the ends 53a,b of the catch arms 52a,b, a spring mechanism 64 (FIG. 8)-located between the second main housing portion 12b and the pushbutton 22-forces the catch arms 52a,b towards the first housing portion 12a. This movement causes the ends 53a,b of the catch arms 52a,b to engage the angled stop members 50a,b. In this position, movement of the lancet holder 36 in the direction of Arrow B due to the drive spring 42 is prevented. After the angled stop members 50a,b have been engaged, the user releases the movable housing 14 and allows the now compressed secondary spring 46 to force the movable housing 14 back to its initial position adjacent the main housing 12, as illustrated in FIG. 6. The lancing device 10 is now in its cocked position, wherein the drive spring 42 is substantially compressed, while the secondary spring 46 is substantially decompressed.

The lancet holder 36 is guided between its resting and cocked positions by a guide rib 56 (FIG. 9) formed on a portion of the lancet holder 36. The guide rib 56 rides within a groove 58 formed between a pair of raised guide rails 60a,b formed in an interior portion of the first main housing 12a.

To perform a puncture on a test subject's skin, the end cap 18 is attached to the lancing device 10. The lancet holder 36 may be in the cocked position at the time the end cap 18 is attached or may be cocked once the end cap 18 has been removably attached to the endcap support 16. The end cap 18 is then placed firmly against the skin where the puncture is to be made, and the pushbutton 22 is depressed. Depressing the pushbutton 22 causes the catch arms 52a,b (FIG. 8)-integrally formed with the bottom of the pushbutton 22-to move toward the first main housing portion 12a away from the lancet holder 36. Thus, the lancet holder 36 is no longer prevented from moving in the direction of Arrow B by the contact of the ends 53a,b of the catch arms 52a,b with the angled stop members 50a,b of the lancet holder 36. The spring mechanism 64 (FIG. 8)-for example, an elastically deformable foam material-is disposed between the pushbutton 22 and a portion of the main housing 12 to bias the pushbutton 22 to its non-actuated position.

Upon release of the lancet holder 36 as described above, the drive spring 42 will force the lancet holder 36 in the direction of Arrow B until the sharp point of the lance 34 (FIG. 3) passes through the aperture 24 in the end cap 18 to make the puncture. As the lancet holder 36 moves in the direction of Arrow B, the attached shaft 38 also moves in the direction of Arrow B. The retainer 40 of the shaft 38 causes the secondary spring 46 to compress as the lancet holder 36 moves to the puncture position. Eventually, the return force of the compressed secondary spring 46 becomes greater than the puncture force of the drive spring 42. At this point, the secondary spring's 46 return force causes the lancet holder 36 to change direction and return to its resting position by moving in the direction of Arrow A. Alternatively, in some embodiments a stop member is provided to stop the lancet holder 36 from moving too far in the direction of Arrow B, at which time the secondary spring 46 returns the lancet holder 36 to its resting position.

However, the lancet holder 36 typically moves in the direction of Arrow A further than required to return to its resting position. Thus, slightly recompressing the drive spring 42, which causes the lancet holder 36 to again travel in the direction of Arrow B. As the lancet holder 36 begins to move back in the direction of Arrow B (due to the slight recompression of the drive spring 42), the secondary spring 46 is recompressed. The force required to recompress the secondary spring 46 effectively dampens the movement of the lancet holder 36. Such damping assists inhibiting or preventing the drive spring 42-and its natural tendency to oscillate (due to its being elastically deformable)-from causing a second, unintended skin puncture to be made.

Turning now to FIG. 8, the pushbutton 22 is illustrated according to one embodiment of the present invention. The pushbutton 22 includes a body 62 from which the two catch arms 52a,b extend. Each of the catch arms 52a,b includes an end 53a,b, respectively, opposite from the body 62. Each end 53a,b is adapted to engage an angled stop member 50a,b of the lancet holder 36. A spring mechanism 64, such as an elastically deformable foam, is located on the underside of the body 62. When the pushbutton 22 is incorporated into the lancing device 10, the spring mechanism 64 contacts a portion of the second main housing portion 12b to bias the pushbutton 22 in the non-actuated position, as discussed above with respect to FIGS. 4-6.

Turning now to FIG. 9, a perspective view of the lancet 30 disposed within the lancet holder 36 is illustrated. The lancet 30 is shown with a protective cap 70 that has a portion that is integrally formed with the lancet body 32 and which covers the sharp point of the lance 34. Prior to using the lancing device 10, the lancet body 32 of a new lancet 30 is inserted into the cylindrical aperture disposed in the lancet holder 36, and then the protective cap 70 is twisted off of the lancet assembly 30, in the direction of the Arrow C shown in FIG. 9.

The lancet holder 36 includes the guide rib 56 that is adapted to be inserted into the groove 58 (FIGS. 5-6). The guide rib 56 and groove 58 are adapted to assist in providing a linear puncture of the test subject's skin by the lancet 30. Linear punctures are preferable because they tend to produce a less painful, and faster healing, piercing of the skin.

The structure of the above-described lancing device 10 provides a number of advantages not previously realized by typical lancing devices. For example, the secondary spring 46 is used to both move the movable housing 14 from the cocking position to the resting position as well as to return the lancet holder 36 from its puncture position to its resting position. Thus, the lancing device 10 is fully functional by utilizing only two springs.

The use of two opposing springs allows for the puncture strength to be adjusted merely by adjusting the spring ratio between the drive spring 42 and the secondary spring 46, reducing the need to compute the frictional interaction and mass of the various components of the device. Typically, the spring constant of the drive spring 42 is greater than the spring constant of the secondary spring 46, which causes the secondary spring 46 to initially be compressed by the force provided by the drive spring 42.

The structure of the above-described lancing device 10 also allows for both the drive spring 42 and the secondary spring 46 to remain free floating on the shaft 38. Thus, the need for attaching one or both ends of each spring is eliminated, reducing the cost and time required to manufacture the lancing device 10.

While the present invention has been described with reference to one or more particular embodiments, those skilled in the art will recognize that many changes may be made thereto without departing from the scope of the present invention. Each of these embodiments and obvious variations thereof is contemplated as falling within the scope of the claimed invention, which is set forth in the following claims.

## Claims

1. A lancing device (10), comprising: a main housing (12) having an internal surface enclosing a portion of a lancing mechanism (24), the lancing mechanism (24) including a lancet holder (36) attached to a shaft (38) and a drive spring (42) surrounding a portion of the shaft (38), the drive spring (42) being located between the lancet holder (36) and the internal surface, the lancing mechanism (24) being adapted to move between a resting position, a cocking position, and a puncture position; and a movable housing (14) adjacent the main housing (12), the movable housing (14) being adapted to move from a resting position to a cocking position, the moveable housing (14) having an internal surface enclosing a portion of the shaft (38) of the lancing mechanism (24), the enclosed portion of the shaft (38) having a retainer (40) and a secondary spring (46) surrounding at least a section of the shaft (38), the secondary spring (46) being located between the retainer (40) and the internal surface of the movable housing (14),
**characterized in that** the secondary spring (46) is adapted to move the movable housing (14) from the cocking position to the resting position, the secondary spring (46) being further adapted to move the lancing mechanism (24) from the puncture position to the resting position.

2. The lancing device (10) of claim1, wherein the secondary spring (46) has a spring constant less than the spring constant of the drive spring (42).

3. The lancing device (10) of claim1, wherein the drive spring (42) is not attached to the lancet holder (36) or the internal surface of the main housing (12).

4. The lancing device (10) of claim 1, wherein the secondary spring (46) is not attached to the retainer (40) of the shaft (38) or the internal surface of the movable housing (14).

5. The lancing device (10) of claim 1, wherein neither the drive spring (42) nor the secondary spring (46) are attached to any component of the lancing mechanism (10).

6. The lancing device (10) of claim1, wherein the secondary spring surrounds the entirety of the portion of the shaft (38) enclosed within the movable housing (14).

## Patentansprüche

1. Lanzettenvorrichtung (10), umfassend ein Hauptgehäuse (12) mit einer einen Abschnitt eines Lanziermechanismus (24) umschließenden Innenfläche, wobei der Lanziermechanismus (24) einen an einer Welle (38) angebrachten Lanzettenhalter (36) und eine Antriebsfeder (42) umfasst, die einen Abschnitt der Welle (38) umgibt, wobei die Antriebsfeder (42) zwischen dem Lanzettenhalter (36) und der Innenfläche angeordnet ist, wobei der Lanziermechanismus (24) ausgebildet ist, um zwischen einer Ruheposition, einer spannenden Position und einer Punktierposition bewegbar zu sein; und ein an das Hauptgehäuse (12) angrenzendes bewegbares Gehäuse (14), wobei das bewegbare Gehäuse (14) ausgebildet ist, um sich aus einer Ruheposition zu einer spannenden Position zu bewegen, wobei das bewegbare Gehäuse (14) eine Innenfläche aufweist, die einen Abschnitt der Welle (38) des Lanziermechanismus (24) umgibt, wobei der umgebene Abschnitt der Welle (38) eine Halterung (40) und eine sekundäre Feder (46) aufweist, die zumindest einen Abschnitt der Welle (38) umgibt, wobei die sekundäre Feder (46) zwischen der Halterung (40) und der Innenfläche des bewegbaren Gehäuses (14) liegt,
**dadurch gekennzeichnet, dass** die sekundäre Feder (46) ausgebildet ist, um das bewegbare Gehäuse (14) aus der spannenden Position zur Ruheposition zu bewegen, wobei die sekundäre Feder (46) ferner ausgebildet ist, um den Lanziermechanismus (24) aus der Punktierposition zur Ruheposition zu bewegen.

2. Lanzettenvorrichtung (10) nach Anspruch 1, worin die sekundäre Feder (46) eine Federkonstante aufweist, die niedriger als die Federkonstante der Antriebsfeder (42) ist.

3. Lanzettenvorrichtung (10) nach Anspruch 1, worin die Antriebsfeder (42) nicht an den Lanzettenhalter (36) oder die Innenfläche des Hauptgehäuses (12) angebracht ist.

4. Lanzettenvorrichtung (10) nach Anspruch 1, worin die sekundäre Feder (46) nicht an die Halterung (40) der Welle (38) oder die Innenfläche des bewegbaren Gehäuses (14) angebracht ist.

5. Lanzettenvorrichtung (10) nach Anspruch 1, worin weder die Antriebsfeder (42) noch die sekundäre Feder (46) an irgendeine Komponente des Lanziermechanismus (10) angebracht sind.

6. Lanzettenvorrichtung (10) nach Anspruch 1, worin die sekundäre Feder den gesamten Abschnitt der innerhalb des bewegbaren Gehäuses (14) umgebenen Welle (38) umgibt.

## Revendications

1. Autopiqueur (10), comprenant un boîtier principal (12) ayant une surface interne enfermant une partie d'un mécanisme autopiqueur (24), le mécanisme autopiqueur (24) comprenant un porte-lancette (36) fixé à un arbre (38) et un ressort d'entraînement (42) entourant une partie de l'arbre (38), le ressort d'entraînement (42) étant situé entre le porte-lancette (36) et la surface interne, le mécanisme autopiqueur (24) étant apte à se déplacer entre une position de repos, une position d'armement, et une position de perforation ; et un boîtier mobile (14) adjacent au boîtier principal (12), le boîtier mobile (14) étant apte à se déplacer d'une position de repos à une position d'armement, le boîtier mobile (14) ayant une surface interne enfermant une partie de l'arbre (38) du mécanisme autopiqueur (24), la partie enfermée de l'arbre (38) ayant un élément de retenue (40) et un ressort secondaire (46) entourant au moins une section de l'arbre (38), le ressort secondaire (46) étant situé entre l'élément de retenue (40) et la surface interne du boîtier mobile (14),
**caractérisé en ce que** le ressort secondaire (46) est apte à déplacer le boîtier mobile (14) de la position d'armement à la position de repos, le ressort secondaire (46) étant en outre apte à déplacer le mécanisme autopiqueur (24) de la position de perforation à la position de repos.

2. Autopiqueur (10) selon la revendication 1, dans lequel le ressort secondaire (46) a une constante de rappel inférieure à la constante de rappel du ressort d'entraînement (42).

3. Autopiqueur (10) selon la revendication 1, dans lequel le ressort d'entraînement (42) n'est pas fixé au porte-lancette (36) ou à la surface interne du boîtier principal (12).

4. Autopiqueur (10) selon la revendication 1, dans lequel le ressort secondaire (46) n'est pas fixé à l'élément de retenue (40) de l'arbre (38) ou la surface interne du boîtier mobile (14).

5. Autopiqueur (10) selon la revendication 1, dans lequel ni le ressort d'entraînement (42), ni le ressort secondaire (46) n'est fixé à une quelconque pièce du mécanisme autopiqueur (10).

6. Autopiqueur (10) selon la revendication 1, dans lequel le ressort secondaire entoure l'intégralité de la partie de l'arbre (38) enfermée au sein du boîtier mobile (14).
